(19) Europäisches Patentamt
European Patent Office
Office européen des brevets

(11) **EP 4 112 039 A1**

(12) **EUROPEAN PATENT APPLICATION**

(43) Date of publication:
**04.01.2023 Bulletin 2023/01**

(21) Application number: **22179970.3**

(22) Date of filing: **20.06.2022**

(51) International Patent Classification (IPC):
**A61K 8/365** (2006.01)    **A61K 8/34** (2006.01)
**A61K 8/42** (2006.01)    **A61Q 19/00** (2006.01)
**A61Q 19/10** (2006.01)

(52) Cooperative Patent Classification (CPC):
**A61K 8/365; A61K 8/345; A61K 8/42;**
**A61Q 19/005; A61Q 19/007; A61Q 19/10**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.07.2021 US 202163217473 P**
**02.12.2021 EP 21212094**

(71) Applicants:
• **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO RS SE SI SK SM**
• **Unilever Global IP Limited**
**Wirral, Merseyside CH62 4ZD (GB)**
Designated Contracting States:
**CY DE GB IE IT MT TR**

(72) Inventors:
• **GENCARELLI, Suzanne Lynn**
**6708 WH Wageningen (NL)**
• **MICHAUD, Catherine Mary**
**6708 WH Wageningen (NL)**
• **MCCOLGAN, Marie Lynn**
**6708 WH Wageningen (NL)**
• **SUBRAMANIAN, Vivek**
**6708 WH Wageningen (NL)**
• **YOKUBINAS, Leonora H.**
**6708 WH Wageningen (NL)**
• **HERMANSON, Kevin, David**
**6708 WH Wageningen (NL)**

(74) Representative: **James, Helen Sarah**
**Unilever PLC**
**Unilever Patent Group**
**Bronland 14**
**6708 WH Wageningen (NL)**

(54) **COSMETIC COMPOSITION CONTAINING A POLYOL, AN ORGANIC ACID AND A VITAMIN B5 COMPOUND**

(57)    Disclosed herein is use of a polyol in combination with organic acid or derivative thereof and a vitamin $B_5$ compound for protecting and/or supporting protein and lipid integrity in the skin barrier. Disclosed herein is also use of polyol, organic acid or derivative thereof, and a vitamin $B_5$ compound for promoting the skin's inherent production of amino acid precursors.

EP 4 112 039 A1

## Description

Technical Field

Disclosed herein are personal care compositions. More particularly, disclosed herein is use of cosmetic compositions containing polyol, organic acid or derivative thereof, and a vitamin $B_5$ compound to protect and support the integrity of proteins and lipids of the skin barrier, as well as to promote the production of amino acid precursors in the skin.

Background

Macromolecules, namely proteins, lipids and carbohydrates, are essential cellular components in biological systems such as the large human organ known as the skin. As a human being's first line of defense as a physical barrier to all external stressors such as UV radiation and pollution, the integrity of the skin barrier is often challenged. It is known that changes and/or compromises in lipid or protein composition may lead to a disturbed skin barrier, which may later contribute negatively to dryness and irritation as well as in the pathogenesis of various other skin conditions.

Surfactants are commonly found in most personal care compositions topically applied to the skin, including wash-off and leave-on compositions. Surfactants pose a challenge of a chemical nature to the integrity of the skin barrier as a whole, including the proteins and lipids that comprise it. While all surfactants interact with lipids and proteins, the intensity of such interactions differ from harsher surfactants to gentler ones, where a stronger interaction (due to the chemical and/or physical properties of the particular surfactant choice) may likely contribute to increased skin irritation. Minimizing surfactant induced damage to stratum corneum proteins and lipids is the first step towards providing moisturization benefits and leaving the skin barrier intact to properly perform its biological functions.

The present inventors have found that use of a polyol in combination with a vitamin $B_5$ compound and an organic acid or a derivative thereof in a cosmetic composition comprising at least one surfactant can cleanse skin while simultaneously providing protection and support for the integrity of proteins and lipids inherently found within the skin barrier.

It has also been determined that use of a polyol, organic acid or derivative thereof, and a vitamin $B_5$ compound in a cosmetic composition can promote the production of necessary amino acid precursors required for subsequent amino acid production in the skin.

Amino acids are the building blocks of protein, which are naturally abundant in the human skin barrier and are essential in keeping the skin strong and healthy. Humans beings are capable of synthesizing certain amino acids referred to as "nonessential amino acids" through inherent biological pathways. The citric acid cycle, otherwise known as the TCA (tricarboxylic acid cycle) or the Krebs cycle, is a major metabolic pathway that provides several amino acid precursors for internal production. The organic acid citric acid in its ionized form of citrate at biological pH is consumed as a part of the TCA cycle and regenerated as the cycle progresses through the series of chemical reactions which define this pathway. The carbon skeletons of many nonessential amino acids (i.e., aspartate, asparagine, glutamine, proline and arginine) are generated from intermediates produced in the TCA cycle. Vitamins such as vitamin $B_5$ (i.e., pantothenic acid or derivatives thereof such as panthenol) also power amino acid precursor production in the TCA cycle in their enzyme form (e.g., coenzyme A).

Without intending to be bound or limited by theory, it is further believed that polyols, including glycerin, due to their humectant function, render the micro-environment in skin cells to become more ideal for amino acids to combine into peptides and proteins. By hydrating the skin, polyols draws and locks in water in addition to other compounds beneficial for a healthy skin barrier, thereby allow the skin to be in optimal condition to function and produce the needed amino acids for skin barrier maintenance and repair.

Therefore, the present inventors have found that use of a polyol such as glycerin in combination with citric acid and a vitamin $B_5$ compound such as panthenol in a cosmetic composition ensures that the skin produces the necessary amino acid precursors required for subsequent amino acid production. Ensuring that amino acids precursors, the building blocks for proteins, are replenished and readily bioavailable allows for protein production to occur downstream.

Additional Information

Efforts have been disclosed for incorporating organic acid, polyol and a vitamin $B_5$ compound in topical cosmetic compositions.

U.S. Patent Application Publication No. 2003/0211068 discloses a cosmetic method for providing improved skin hydration comprising the steps of topically applying to the skin a protease enzyme and a skin care active selected from a vitamin $B_3$ component, vitamin E acetate, panthenol and mixtures thereof.

U.S. Patent No. 8,628,758 relates to a process for making up the lips, comprising at least one step of placing in contact with the lips: at least one water-soluble organic acid, at least one alkali metal or alkaline-earth metal carbonate or bicarbonate, and an aqueous medium.

U.S. Patent No. 10,973,749 describes a cosmetic composition for delivering high amounts of ascorbic acid and ceramides to the skin, wherein the composition includes ascorbic acid, ceramides, panthenol, monoalcohols having from 2 to 6 carbon atoms, polyol, fatty compounds, emulsifiers, cationic surfactants, and water.

PCT Publication No. WO 1999/47114 relates to a skin moisturizing composition comprising a vitamin B3 compound and a ceramide pathway intermediate or precursors thereof.

PCT Publication No. WO 2021/082683 discloses a two-part composition for caring for keratin materials comprising: a) an anhydrous part containing at least one water-soluble active ingredient; and b) a hydrous part containing: i) a xanthan gum, and ii) at least one $C_{2-6}$ polyol.

None of the additional information above describes the particular combination of polyol, organic acid or derivative thereof, and a vitamin $B_5$ compound to be used to protect and support the proteins and lipids of the stratum corneum, as well as promote the skin's inherent amino acid production via a topically applied cosmetic composition as disclosed herein.

## Summary

The present inventors have found use of polyol, organic acid or derivative thereof, and a vitamin $B_5$ compound to cleanse skin and provide protection and support for the proteins and lipids inherently found within the skin barrier.

Accordingly, in a first aspect, disclosed herein is the use of a polyol in combination with organic acid such as citric acid or derivative thereof and a vitamin $B_5$ compound for protecting and/or supporting protein and lipid integrity in the skin barrier.

In a second aspect, disclosed is the use of polyol, organic acid or a derivative thereof, and a vitamin $B_5$ compound for promoting the skin's inherent production of amino acid precursors.

In a third aspect, disclosed herein is a cosmetic composition comprising: (a) one or more of a polyol; (b) one or more of organic acid or derivative thereof; and (c) one or more of a vitamin $B_5$ selected from the group consisting of pantothenic acid, pantothenic acid salt, panthenol, panthenol derivative, pantolactone, and mixtures thereof.

In a fourth aspect, disclosed is a method of cleansing the skin without compromising the integrity of the skin's proteins and/or lipids comprising the steps of:

(a) contacting the skin with a cosmetic composition comprising polyol, organic acid or derivative thereof, and a vitamin $B_5$ compound; and
(b) washing the composition off the skin.

All other aspects of the present cosmetic compositions will become more readily apparent upon considering the detailed description and examples which follow.

For the avoidance of doubt, the term "comprising" is meant not to be limiting to any stated elements but rather to encompass non-specified elements of major or minor functional importance. Therefore, the listed steps, elements or options need not be exhaustive. Whenever the words "including" or "having" are used, these terms are meant to be equivalent to "comprising" as defined above.

The term "skin" as used herein includes the skin on the face, neck, chest, back, arms, axilla, buttocks, hands, legs and scalp. Skin benefit agent, as used herein, is meant to include a component that improves a facial or body characteristic after topical application like a skin characteristic and/or benefits the same wherein the skin benefit agent can be, and preferably is, a skin benefit agent in a cream, pump or aerosol spray, serum, lotion, balm, deodorant, gel or wash composition. In an embodiment, the cosmetic composition is a wash-off composition. In another embodiment, the cosmetic composition is a leave-on composition. The term "leave-on" as used with reference to compositions herein means a composition that is applied to or rubbed on the skin and left thereon. The term "derivative(s)" as used with reference to ingredients herein refers to functional group substitutions on the compound. The term "functional group," as used herein, includes an acyl, hydroxy, alkoxy, carboxamide (amide), carboxyl, or carboalkoxy (ester) substituent, on the compound. Ambient temperature, as used herein, refers to a temperature of 20 to 25°C.

As used herein, protecting and supporting the integrity of skin proteins and lipids refers to effects, but in a nonlimiting manner, such as decreasing solubilization of skin proteins and lipids, optimizing mildness of the impact of surfactants when topically applied to the skin, increasing cohesivity of the skin barrier, and providing nourishment and enhanced depositing of building blocks for compounds required for skin barrier maintenance and/or repair.

Except in the examples, or where otherwise explicitly indicated, all numbers in this description indicating amounts of material are to be understood as modified by the word "about." All amounts are by total weight of the composition, unless otherwise specified.

Detailed Description

The inventive compositions contain a polyol. The polyol compounds comprised in the cosmetic compositions disclosed herein are polyhydric alcohol-type materials. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propanediol, propoxylated glycerol and mixtures thereof. The preferred embodiment includes glycerin, butylene glycol, sorbitol, propanediol or a mixture thereof. More preferably, the polyol is glycerin, propanediol, or mixtures thereof. The amount of polyol employed may range anywhere from 0.1 to 50%, preferably, 0.25 to 40%, more preferably, 0.5 to 30% by weight of the cosmetic composition. It is also preferred that polyol is 0.75 to 20%, still preferably, 1 to 15% by weight of the cosmetic composition.

Among the organic acids desirable to achieve the use disclosed herein, any organic acid commonly employed in cosmetic or pharmaceutical compositions may be selected. Preferably, the organic acid is a $C_2$-$C_{22}$ carboxylic acid. It is also preferred that the organic acid is soluble in water. Desirable organic acids for use in the disclosed cosmetic compositions may comprise, in a nonlimiting manner, adipic acid, ascorbic acid, aspartic acid, benzoic acid, cinnamic acid, citric acid, fumaric acid, glutamic acid, glutaric acid, glycolic acid, hydroxybenzoic acid, hydroxycinnamic acid, lactic acid, maleic acid, malic acid, malonic acid, nicotinic acid, phenylacetic acid, pimelic acid, salicylic acid, sorbic acid, tartaric acid, tartronic acid, tropic acid, and derivatives or mixtures thereof. It is particularly preferred that the organic acid is citric acid, lactic acid, or derivatives (e.g., citrate) or mixtures thereof. It is preferable that the organic acid be anhydrous citric acid, citric acid monohydrate, or a mixture thereof. The cosmetic composition comprises 0.001 to 10%, preferably, 0.002 to 8%, more preferably, 0.003 to 6% organic acid by weight of the cosmetic composition.

It is also desirable to include a vitamin $B_5$ compound selected from the group consisting of pantothenic acid, salts of pantothenic acid (e.g., sodium pantothenate, calcium pantothenate), panthenol, derivatives of panthenol (e.g., D-panthenol (dexpanthenol), DL-panthenol, panthenol thioesters), pantolactone, and mixtures thereof. The alcohol analogue of pantothenic acid, also known as vitamin $B_5$, is panthenol, which is converted internally in humans to pantothenic acid. It is preferred that the vitamin $B_5$ compound is D-panthenol, DL-panthenol, or a mixture thereof. It is especially preferred that the vitamin $B_5$ compound is D-panthenol. The amount of vitamin $B_5$ compound employed may range anywhere from 0.001 to 9%, preferably, 0.005 to 7%, and, more preferably, 0.01 to 5% by weight of the cosmetic composition. It is also preferred that the cosmetic composition comprises 0.01 to 3% vitamin $B_5$ compound by weight of the cosmetic composition.

Now it has been determined that use of one or more of polyol, one or more of organic acid or a derivative thereof, and one or more of a vitamin $B_5$ compound in a surfactant-containing cosmetic composition cleanses skin while protecting and/or supporting the integrity of proteins and lipids in the skin where surfactants would normally induce disruption and/or damage. The use is preferably non-therapeutic in nature, more preferably cosmetic in nature.

It has also been determined that use of one or more of polyol, one or more of organic acid or a derivative thereof, and one or more of a vitamin $B_5$ compound in a cosmetic composition may promote the skin's inherent amino acid production via topical application of the disclosed cosmetic composition. The use is preferably non-therapeutic in nature, more preferably cosmetic in nature.

Cosmetic compositions comprising polyol, organic acid or derivative thereof, and a vitamin $B_5$ compound may further comprise a cosmetically acceptable carrier. Desirable carriers may be chosen based on the desired format, preferably, the format is a leave-on composition or a cleansing, wash-off composition.

Leave-on composition

Where the cosmetic composition is in the form of a leave-on composition, the composition may comprise a surfactant that is an emulsifier. The emulsifier may be selected from the group consisting of anionic, nonionic, cationic and amphoteric surfactants. Particularly preferred nonionic surfactants are those with a $C_{10}$-$C_{20}$ fatty alcohol or acid hydrophobe condensed with from about 2 to about 100 moles of ethylene oxide or propylene oxide per mole of hydrophobe; $C_2$-$C_{10}$ alkyl phenols condensed with from 2 to 20 moles of alkylene oxide; mono- and di-fatty acid esters of ethylene glycol; fatty acid monoglyceride; sorbitan, mono- and di- $C_8$-$C_{20}$ fatty acids; and polyoxyethylene sorbitan as well as combinations thereof. Alkyl polyglycosides and saccharide fatty amides (e.g. methyl gluconamides) are also desriable nonionic emulsifiers. Still other preferred nonionic surfactants include glyceryl stearate, glycol stearate and stearamide AMP.

Preferred anionic emulsifiers include alkyl ether sulfate and sulfonates, alkyl sulfates and sulfonates, alkylbenzene sulfonates, alkyl and dialkyl sulfosuccinates, $C_8$-$C_{20}$ acyl isethionates, $C_8$-$C_{20}$ acyl methyl isethionates, $C_8$-$C_{20}$ acyl methyl taurates, $C_8$-$C_{20}$ alkyl ether phosphates, alkyl ether carboxylates and combinations thereof.

Cationic emulsifiers that may be used include, for example, palmitamidopropyltrimonium chloride, distearyldimonium chloride, diester quaternary ammonium compounds (e.g. distearoylethyl dimonium chloride) and mixtures thereof.

The cosmetic compositions may also comprise a cosmetically acceptable vehicle to act as a cosmetically acceptable diluent, dispersant or carrier for the skin benefit agents in the composition, so as to facilitate its distribution when the composition is applied to the skin. This cosmetically acceptable vehicle may be aqueous, anhydrous or an emulsion. Oily carriers in the presence of water and an emulsifier will form emulsion systems as carriers. Preferably, the compositions are aqueous or an emulsion, especially water-in-oil or oil-in-water emulsion, preferentially oil-in-water emulsion. The cosmetic compositions ordinarily will be in but are not limited to cream or lotion form.

Desirable carriers employed include water, sea water, rosewater (e.g. *Rosa Damascena* flower Water), tea (e.g. *Camellia Sinensis* Leaf Water), *Aloe Barbadensis* (aloe vera) leaf juice, witch hazel (e.g. *Hamamelis Virginiana* extract), lavender water (e.g. *Lavendula Angustifolia* flower water), grape water (e.g. *Vitis Vinifera* fruit water and *Vitis Vinifera* juice). In the most preferred embodiment, water is the carrier. Amounts of water may preferably range from 5 to 85%, more preferably, from 15 to 75%, and most preferably, from 30 to 70%, based on the weight of the cosmetic composition and including all ranges subsumed therein. The cosmetically acceptable vehicle can, in the absence of other cosmetic adjuncts, form the balance of the composition. Besides water, desirable carrier classes include, but are not limited to, silicones, fatty acids, hydrocarbons, triglycerides, and waxes.

Silicones may be categorized into the volatile and nonvolatile variety. Amounts may range, for example, from 0.01 to 8%, more preferably from 0.1 to 6% by weight of the composition. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Nonvolatile silicones useful in this composition include polyalkyl siloxanes, polyalkylaryl siloxanes and polyether siloxane copolymers. The essentially nonvolatile polyalkyl siloxanes useful herein include, for example, polydimethyl siloxanes with viscosities from about $5 \times 10^{-6}$ to $0.1\ m^2/s$ at 25°C. Emulsifying and non-emulsifying silicone elastomers are also desirable for use in the inventive cosmetic composition.

Fatty acids may also be useful carriers. The term "fatty" refers to carbon chain lengths ranging from 10 to 30 carbon atoms. Illustrative of this category are pelargonic, lauric, myristic, palmitic, stearic, isostearic, hydroxystearic, oleic, linoleic, ricinoleic, arachidic, behenic, erucic acids and combinations thereof. When used, from 0.01 to 5% by weight fatty acid is present in the composition.

Waxes and wax esters are also desirable for use in the cosmetic compositions. These waxes include animal-derived waxes or those from animal byproducts (e.g., beeswax, spermaceti wax, Chinese wax, wool wax, shellac wax), plant-derived waxes (e.g., tribehenin wax, carnauba wax, candelilla wax, bayberry wax, jojoba wax, orange wax, rice bran wax, sunflower wax, castor wax, soy wax), mineral waxes (e.g., montan wax, ceresin wax, ozokerite) and synthetic derivatives of natural waxes. The amount of wax and wax esters employed may range from 0.01 to 10%, preferably from 0.1 to 8% by weight of the composition.

Triglycerides are another group of materials useful as carriers. Illustrative but not limiting examples are sunflower seed oil, cotton oil, canola oil, grapeseed oil, soybean oil, castor oil, borage oil, olive oil, shea butter, jojoba oil and mixtures thereof. Mono- and di-glycerides may also be useful. Illustrative of these categories are glyceryl monostearate and glyceryl distearate. Amounts of triglycerides may range from 0.01 to 10%, preferably from 0.1 to 8% by weight of the composition.

Hydrocarbons may be optionally included in the cosmetic composition. Desirable hydrocarbons may include mineral oil, petrolatum and polyalpha-olefins. Examples of preferred volatile hydrocarbons include polydecanes such as isodecane and isododecane and the $C_7$-$C_8$ through $C_{12}$-$C_{15}$ isoparaffins.

It is within the scope of the cosmetic compositions to optionally include sunscreens and photostabilizers. The sunscreens and photostabilizers that may be used in the cosmetic compositions include such materials as octylmethoxycinnamate (OMK), ethylhexyl salicylate, phenylbenzimidazole sulfonic acid (Ensulizole), ethylhexyl p-methoxycinnamate, available as Parsol MCX®, Avobenzene (butyl methoxydibenzoylmethane), available as Parsol 1789® and benzophenone-3, also known as oxybenzone. Inorganic sunscreen actives may be employed such as microfine titanium dioxide (preferably, with a particle diameter of less than 150nm, and, most preferably, less than 100nm) and zinc oxide may be used, polyethylene and various other polymers are also desirable sunscreens. Other sunscreens desirable for use include p-aminobenzoic acid (PABA), octyldimethyl-PABA, 2-ethoxyethyl p-methoxy cinnamate, benzophenone-1, benzophenone-2, benzophenone-6, benzophenone-8, benzophenone-9, benzophenone-12, homomethyl salicylate, menthyl anthranilate, benzophenone-4, triethanolamine salicylate, terephthalylidene dicamphor sulfonic acid, bisoctriazole, bisethylhexyloxyphenol methoxyphenyl triazine, bisdisulizole disodium, diometriazole trisiloxane, octyltriazone, iscotrizinol, polysilicone-15, isopentenyl-4-methoxycinnamate, mixtures thereof or the like. Also desirable for use is octocrylene. Amounts of the sunscreen or photostabilizing agents when present may generally range from 0.1 to 20%, preferably, from 0.5 to 15%, optimally, from 0.75 to 10% by weight of the cosmetic composition.

Wash-off composition

Where the cosmetic compositions disclosed herein are in the form of wash-off compositions, preferably cleansing compositions, the composition can include a surfactant, specifically, the cosmetic composition can include 25% to 35% by weight of the surfactant. The surfactant can be present in an amount of greater than 25% by weight and less than 35% by weight. The surfactant can be present in an amount of 26% to 32% by weight.

The cosmetic, wash-off composition can include a co-surfactant, specifically, the cosmetic composition can include 1.5% to 5% by weight of the co-surfactant. The co-surfactant can be present in an amount of greater than or equal to 1.5% by weight and less than or equal to 5% by weight. The co-surfactant can be present in an amount of 2.0% to 4% by weight, for example, 2.5% to 3.5% by weight.

The surfactant and/or co-surfactant can be selected from an anionic surfactant, a zwitterionic surfactant, an amphoteric surfactant, or a combination thereof. The discussion which follows refers to the surfactant, the co-surfactant, or the surfactant and the co-surfactant. The surfactant and/or co-surfactant can contain $C_8$-$C_{18}$ alkyl groups, for example, $C_{12}$-$C_{16}$ alkyl groups, for example, $C_{10}$-$C_{14}$ alkyl groups, or mixtures thereof. For example, the surfactant and/or co-surfactant can contain $C_{10}$ alkyl groups, $C_{12}$ alkyl groups, $C_{14}$ alkyl groups, or any combination thereof.

When present, the anionic surfactant used can include aliphatic sulfonates, such as a primary alkane (e.g., $C_8$-$C_{22}$) sulfonate, primary alkane (e.g., $C_8$-$C_{22}$) disulfonate, $C_8$-$C_{22}$ alkene sulfonate, $C_8$-$C_{22}$ hydroxyalkane sulfonate or alkyl glyceryl ether sulfonate (AGS); or aromatic sulfonates such as alkyl benzene sulfonate. The anionic surfactant may also be an alkyl sulfate (e.g., $C_{12}$-$C_{18}$ alkyl sulfate) or alkyl ether sulfate (including alkyl glyceryl ether sulfates). Among the alkyl ether sulfates are those having the formula:

$$RO(CH_2CH_2O)_nSO_3M$$

wherein R is an alkyl or alkenyl having 8 to 18 carbons, preferably, 12 to 18 carbons, n has an average value of at least 1.0, preferably, less than 5, and, most preferably, 1 to 4, and M is a solubilizing cation such as sodium, potassium, ammonium or substituted ammonium.

The anionic surfactant may also be alkyl sulfosuccinates (including mono- and dialkyl, e.g., $C_6$-$C_{22}$ sulfosuccinates); alkyl and acyl taurates (often methyl taurates), alkyl and acyl sarcosinates, sulfoacetates, $C_8$-$C_{22}$ alkyl phosphates and phosphonates, alkyl phosphate esters and alkoxyl alkyl phosphate esters, acyl lactates, $C_8$-$C_{22}$ monoalkyl succinates and maleates, sulphoacetates, alkyl glucosides and acyl isethionates, and the like.

Sulfosuccinates may be monoalkyl sulfosuccinates having the formula:

$$R^1OC(O)CH_2CH(SO_3M)CO_2M;$$

and amide-MEA sulfosuccinates of the formula:

$$R^1CONHCH_2CH_2OC(O)CH_2CH(SO_3M)CO_2M$$

wherein $R^1$ ranges from $C_8$-$C_{22}$ alkyl.

Sarcosinates are generally indicated by the formula:

$$R^2CON(CH_3)CH_2CO_2M, \text{ wherein } R^2 \text{ ranges from } C_8\text{-}C_{20} \text{ alkyl.}$$

Taurates are generally identified by formula:

$$R^3CONR^4CH_2CH_2SO_3M$$

wherein $R^3$ is a $C_8$-$C_{20}$ alkyl, $R^4$ is a $C_1$-$C_4$ alkyl.

M is a solubilizing cation as previously described.

The cosmetic composition disclosed herein may contain $C_8$-$C_{18}$ acyl isethionates. These esters are prepared by a reaction between alkali metal isethionate with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms.

The acyl isethionate may be an alkoxylated isethionate such as is described in Ilardi et al., U.S. Pat. No. 5,393,466, entitled "Fatty Acid Esters of Polyalkoxylated isethonic acid; issued Feb. 28, 1995; hereby incorporated by reference. This compound has the general formula:

$$R^5C\text{-}(O)O\text{-}C(X)H\text{-}C(Y)H\text{-}(OCH_2\text{-}CH_2)_m\text{-}SO_3M$$

wherein $R^5$ is an alkyl group having 8 to 18 carbons, m is an integer from 1 to 4, X and Y are each independently hydrogen or an alkyl group having 1 to 4 carbons and M is a solubilizing cation as previously described.

In an aspect of the cosmetic composition, the anionic surfactant used is 2-acrylamido-2-methylpropane sulfonic acid, ammonium lauryl sulfate, ammonium perfluorononanoate, potassium lauryl sulfate, sodium alkyl sulfate, sodium dodecyl sulfate, sodium laurate, sodium laureth sulfate, sodium lauroyl sarcosinate, sodium stearate, sodium sulfosuccinate esters, sodium lauroyl isethionate, or a combination thereof. Such anionic surfactants are commercially available from suppliers like Galaxy Surfactants, Clariant, Sino Lion, Stepan Company, and Innospec.

Optionally, amphoteric surfactants can be included in the cosmetic compositions disclosed herein. Amphoteric surfactants (which depending on pH can be zwitterionic) include sodium acyl amphoacetates, sodium acyl amphopropionates, disodium acyl amphodiacetates and disodium acyl amphodipropionates where the acyl (i.e., alkanoyl group) can comprise a $C_7$-$C_{18}$ alkyl portion. Illustrative examples of amphoteric surfactants include sodium lauroamphoacetate, sodium cocoamphoacetate, sodium lauroamphoacetate, or a combination thereof.

As to the zwitterionic surfactants employed in the present cosmetic composition, such surfactants include at least one acid group. Such an acid group may be a carboxylic or a sulphonic acid group. They often include quaternary nitrogen, and therefore, can be quaternary amino acids. They should generally include an alkyl or alkenyl group of 7 to 18 carbon atoms and generally comply with an overall structural formula:

$$R^6\text{-}[\text{-C(O)-NH(CH}_2)_q\text{-}]_r\text{-N}^+(R^7)(R^8)\text{-A-B}$$

where $R^6$ is alkyl or alkenyl of 7 to 18 carbon atoms; $R^7$ and $R^8$ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is $-CO_2-$ or $-SO_3-$.

Desirable zwitterionic surfactants for use in the cosmetic composition disclosed herein and within the above general formula include simple betaines of formula:

$$R^6\text{-N}^+(R^7)(R^8)\text{-CH}_2CO_2{}^-$$

and amido betaines of formula:

$$R^6\text{-CONH(CH}_2)_t\text{-N}^+(R^7)(R^8)\text{-CH}_2CO_2{}^-$$

where t is 2 or 3.

In both formulae $R^6$, $R^7$ and $R^8$ are as defined previously. $R^6$ may, in particular, be a mixture of $C_{12}$ and $C_{14}$ alkyl groups derived from coconut oil so that at least half, preferably, at least three quarters of the groups $R^6$ have 10 to 14 carbon atoms. $R^7$ and $R^8$ are preferably methyl.

A further possibility is that the zwitterionic surfactant is a sulphobetaine of formula:

$$R^6\text{-N}^+(R^7)(R^8)\text{-(CH}_2)_3SO_3{}^- \text{ or}$$

$$R^6\text{-CONH(CH}_2)_u\text{-N}^+(R^7)(R^8)\text{-(CH}_2)_3SO_3{}^-$$

where u is 2 or 3, or variants of these in which $-(CH_2)_3SO_3{}^-$ is replaced by

$$-CH_2C(OH)(H)CH_2SO_3{}^-.$$

In these formulae, $R^6$, $R^7$ and $R^8$ are as previously defined.

Illustrative examples of the zwitterionic surfactants desirable for use include betaines such as lauryl betaine, betaine citrate, cocodimethyl carboxymethyl betaine, cocoamidopropyl betaine, coco alkyldimethyl betaine, and laurylamidopropyl betaine. An additional zwitterionic surfactant suitable for use includes cocamidopropyl sultaine, for example, cocamidopropyl hydroxysultaine. Preferred zwitterionic surfactants include lauryl betaine, betaine citrate, sodium hydroxymethylglycinate, (carboxymethyl) dimethyl-3-[(1-oxododecyl) amino] propylammonium hydroxide, coco alkyldimethyl betaine, (carboxymethyl) dimethyloleylammonium hydroxide, cocoamidopropyl betaine, (carboxymethyl) dimethyloleylammonium hydroxide, cocoamidopropyl betaine, (carboxylatomethyl) dimethyl(octadecyl)ammonium, cocamidopropyl hydroxysultaine, or a combination thereof. Such surfactants are made commercially available from suppliers like Stepan Company, Solvay, Evonik and the like and it is within the scope of the cosmetic compositions disclosed herein to employ mixtures of the aforementioned surfactants.

Nonionic surfactants may optionally be used in the cosmetic composition. When used, nonionic surfactants are typically used at levels as low as 0.5, 1, 1.5 or 2% by weight and at levels as high as 6, 8, 10 or 12% by weight. The nonionic surfactants which may be used include in particular the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl ($C_6$-$C_{22}$) phenols, ethylene oxide condensates, the condensation products of aliphatic ($C_8$-$C_{18}$) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides, and the like.

In an aspect, nonionic surfactants can include fatty acid/alcohol ethoxylates having the following structures a) $HOCH_2(CH_2)_s(CH_2CH_2O)_c\,H$ or b) $HOOC(CH_2)_v(CH_2CH_2O)_d\,H$; where s and v are each independently an integer up to 18; and c and d are each independently an integer from 1 or greater. In an aspect, s and v can be each independently 6 to 18; and c and d can be each independently 1 to 30. Other options for nonionic surfactants include those having the formula $HOOC(CH_2)_i\text{-}CH=CH\text{-}(CH_2)_k(CH_2CH_2O)_z\,H$, where i, k are each independently 5 to 15; and z is 5 to 50. In another aspect, i and k are each independently 6 to 12; and z is 15 to 35.

The nonionic surfactant may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995; which is hereby incorporated by reference or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991; hereby incorporated into the subject application by reference.

Illustrative examples of nonionic surfactants that can optionally be used in the cosmetic compositions disclosed herein include, but are not limited to, polyglycoside, cetyl alcohol, decyl glucoside, lauryl glucoside, octaethylene glycol monododecyl ether, n-octyl beta-d-thioglucopyranoside, octyl glucoside, oleyl alcohol, polysorbate, sorbitan, stearyl alcohol, or a combination thereof.

In an aspect, cationic surfactants may optionally be used in the cosmetic composition of the present application.

One class of cationic surfactants includes heterocyclic ammonium salts such as cetyl or stearyl pyridinium chloride, alkyl amidoethyl pyrrylinodium methyl sulfate, and lapyrium chloride.

Tetra alkyl ammonium salts are another useful class of cationic surfactants for use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and stearyl amidopropyl dimethylamine lactate.

Still other useful cationic surfactants include quaternized hydrolysates of silk, wheat, and keratin proteins, and it is within the scope of the cosmetic composition to use mixtures of the aforementioned cationic surfactants.

If used, cationic surfactants will make up no more than 1.0% by weight of the cosmetic composition. When present, cationic surfactants typically make up from 0.01 to 0.7%, and more typically, from 0.1 to 0.5% by weight of the cosmetic composition, including all ranges subsumed therein.

Particularly preferred surfactants for use in the present cosmetic compositions include wherein the surfactant is cocamidopropyl hydroxysultaine, cocamido sulfosuccinate, sodium lauroyl isethionate, or a combination thereof, most preferably, wherein the surfactant is sodium lauroyl isethionate, or a combination thereof.

Particularly preferred co-surfactants for use in the present cosmetic compositions include cocamidopropyl betaine, sodium methyl cocoyl taurate, sodium cocoyl glycinate, sodium cocoyl glutamate, methyl ester sulfonate, fatty acid ester sulfonate or a combination thereof.

The cosmetic composition additionally comprises 5% to 9% by weight of water, for example greater than or equal to 5% by weight water and less than or equal to 9% by weight water. For example, the cosmetic composition comprises 6% to 8% by weight water.

The cosmetic composition also comprises 50% to 60% by weight of a fatty acid and soap mixture. A ratio of the fatty acid to soap can be 2.3:1 to 1.8:1. The high amount of fatty acid and soap mixture present allows for the amount of surfactant to be greatly reduced as compared to other formulations, without a loss in hedonics with a gain towards skin benefits.

The fatty acid can be selected from lauric acid, myristic acid, palmitic acid, stearic acid, behenic acid, oleic acid, linoleic acid, lanolic acid, isostearic acid, arachidonic acid, hydroxystearic acid, or a combination thereof, preferably, wherein the fatty acid is selected from stearic acid, palmitic acid, or a combination thereof.

The term "soap" is used here in its popular sense, i.e., salts of aliphatic alkane- or alkene monocarboxylic fatty acids preferably having 6 to 22 carbon atoms, and preferably 8 to 18 carbon atoms.

Typical of the soap salts are alkali metal or alkanol ammonium salts of such fatty acids, although other metal salts thereof, e.g., magnesium salts, may also be employed. Sodium, potassium, magnesium, mono-, di- and tri-ethanol ammonium salts of such acids are among the desirable soaps for use herein.

The soap can be a neutralized fatty acid. The neutralized fatty acid can be selected from lauric acid, myristic acid, palmitic acid, stearic acid, behenic acid, oleic acid, linoleic acid, lanolic acid, isostearic acid, arachidonic acid, hydroxystearic acid, or a combination thereof, preferably, wherein the fatty acid is selected from stearic acid, palmitic acid, or a combination thereof.

The soap can comprise a mixture of lauric acid and an acid selected from myristic acid, palmitic acid, stearic acid, behenic acid, oleic acid, linoleic acid, lanolic acid, isostearic acid, arachidonic acid, hydroxystearic acid, or a combination thereof. When lauric acid is used, it can be present in an amount of 80% by weight in the fatty acid and soap mixture, for example, the lauric acid can be present in an amount of 85% by weight in the fatty acid and soap mixture. Lauric acid are generally rich in $C_{12}$ and includes coconut oil and/or palm kernel oil.

Additional additives

Cosmetic compositions as disclosed herein can additionally include various additives including, but not limited to, skin benefit agents, fragrances, sensory modifiers, viscosity modifiers, thickeners, preservatives, pH adjusters, polymers, or a combination thereof. Skin benefit agents desirable for use are meant to include but not be limited to opacifiers, colorants, humectants, emollients, occlusive agents, plant extracts, optical agents, even tone agents, anti-inflammatory agents, anti-acne agents, sunscreens, antioxidants, photostabilizers, wrinkle-reducing agents, desquamation promoters, exfoliating agents, mixtures thereof or the like. Each of these substances may range from 0.05 to 5%, preferably between 0.1 and 3% by weight of the cosmetic composition.

Additional optional skin benefit agents desirable for use include minerals and skin nutrients such as milk; magnesium, calcium, copper, zinc and other metallic components; kojic acid; hydroquinone and arbutin; saccharide isomerate; undecylenoyl phenylalanine; sphingolipids, phospholipids (e.g., phytosphingosine), ceramides (e.g. Ceramide 1, Ceramide 3, Ceramide 3B and Ceramide 6) and pseudoceramides; allantoin; pyroglutamic acid (PCA) salt derivatives including zinc PCA and sodium PCA; petroselinic acid; conjugated linoleic acid; octadecanoic acid; hyaluronic acid and its salt derivatives mixtures thereof and the like. Such skin benefit agents, when used, collectively make up from 0.001 to 12% by weight of the cosmetic composition.

A wide selection of botanical extracts may optionally be included in the cosmetic compositions. The extracts may either be soluble in water or oil, carried in a solvent that is hydrophilic or hydrophobic, respectively. In the preferred embodiment, water or ethanol are the extract solvents. Illustrative examples include those extracted from green tea, yarrow, chamomile, licorice, aloe vera, citrus unshui, willow bark, alfalfa, algae, witch hazel, sage, thyme and rosemary, as well as oils such as those derived from sea buckthorn, moringa, argan, avocado, calendula, algal and marula. Soy extracts may be used and especially when it is desirable to include retinol. Such extracts, when used, are preferably employed in collective amounts ranging from 0.001 to 12% by weight of the cosmetic composition.

Another optional additive desirable for use includes hemp oil with 2.5 to 25% by weight cannabigerol and/or cannabidiol at from 0.5 to 10 percent by weight. When used, such oil makes up from 0.0001 to 12% by weight of the composition, and preferably, from 0.01 to 5% by weight of the cosmetic composition, including all ranges subsumed therein.

The cosmetic compositions may optionally comprise one or more thickening agents, preferably from 0.05 to 10%, more preferably, from 0.1 to 5%, and even more preferably, from 0.25 to 4%, by weight of the cosmetic composition. Useful thickeners include polysaccharides, which comprise of starches, natural/synthetic gums and cellulosics. Desirable starches include tapioca starch, cornstarch, potato starch, aluminum starch octenylsuccinate and sodium hydroxypropyl starch phosphate. Desirable gums include xanthan, sclerotium, pectin, karaya, arabic, agar, guar, carrageenan, alginate and combinations thereof. Desirable cellulosics include hydroxypropyl cellulose, hydroxypropyl methylcellulose, ethylcellulose and sodium carboxy methylcellulose. Synthetic polymers functioning as thickening agents are also desirable for use, including taurate copolymers.

It is desirable to employ any pH modifier known in the field acceptable for use in personal care and/or pharmaceutical products to result in a cosmetic composition possessing a skin-acceptable pH, typically within the range of 4 to 8. In a preferred embodiment, the pH of the present cosmetic compositions is from 4.25 to 7.5, and most preferably, from 5 to 7. Traditional buffers or pH modifiers include common additives such as sodium hydroxide, potassium hydroxide, hydrochloric acid, triethanolamine and aminomethyl propanol. Viscosity of the present cosmetic composition is preferably from about 1,000 to about 120,000 cps, and, most preferably, from about 5,000 to 80,000 cps, taken under conditions of ambient temperature and a shear rate of $1s^{-1}$ with a strain controlled parallel plate rheometer made commercially available from suppliers like T.A. Instruments under the Discovery name. Alternatively, viscosity can also be measured using a Brookfield Viscometer (speed at 20 rpm, spindle 5, heliopath off, for one (1) minute at ambient temperature).

The present cosmetic composition can be formulated as a serum having a viscosity from 1,000 to 4,000 mPas, a lotion having a viscosity from 4,000 to 10,000 mPas, a fluid cream having a viscosity from 10,000 to 20,000 mPas or a cream having a viscosity from 20,000 to 100,000 mPas or above. The present cosmetic composition can also be formulated as a cleansing gel having a viscosity from 2,000 to 20,000 mPas.

Optionally, one or more coloring agent may also be included in the cosmetic compositions disclosed herein. Coloring agents include dyes or pigments of natural or synthetic origin. A dye selected for use may be organic or inorganic and water-soluble or oil-soluble. Nonlimiting examples of water-soluble dyes include, for example, D&C Yellow 8, D&C Yellow 10, D&C Orange 4, D&C Red 6, D&C Red 22, D&C Red 28, D&C Red 33, D&C Green 5 or methylene blue. Oil-soluble dyes include, for example, D&C Red 17, D&C Green 6, β-carotene, D&C Violet 2, D&C Yellow 11, D&C Orange 5 and quinoline yellow. Pigments desirable for use are specially chosen from the mineral pigments, organic pigments, lakes and mixtures thereof as known in the art. Illustrative but nonlimiting examples include mineral pigments such as titanium dioxide, ochres (yellow ochre, red ochre, brown ochre, iron hydroxide), metal oxides (zinc oxide, zirconium oxide, iron oxide, cerium oxide, chromium oxide), manganese violet, ultramarine blue, chromium hydrate and ferric blue, or metal powders (aluminum, bronze or copper powder); organic pigments such as nitroso, nitro, azo, phthalocyanine, diazine, xanthene, pyrene, quinoline, anthraquinone, triphenylmethane, fluorane, quinacridone, metal complex, isoindolinone, isoindoline, perinone, perylene, diketopyrrolopyrrole, indigo, thioindigo, dioxazine, triphenylmethane and quinophthalone compounds; and lakes (particularly those deriving from calcium, barium, aluminum and strontium salts) such as D&C Red No. 6 Barium Lake, D&C Red No. 7 Calcium Lake, D&C Red No. 27 Aluminum Lake, D&C Red No. 28 Aluminum Lake, D&C Red No. 33 Aluminum Lake, FD&C Red No. 40 Aluminum Lake, FD&C Yellow No. 5 Aluminum Lake, FD&C Yellow No. 6 Aluminum Lake, D&C Yellow No. 10 Aluminum Lake, D&C Orange No.5 Aluminum Lake and F&D Blue No. 1 Aluminum Lake. Nacres, such as natural mica coated with a metal oxide, bismuth oxychloride or a natural pigment, are also desirable to be included in the inventive composition. Other coloring agents desirable for use include chalk, activated charcoal and carbon black. Cosmetic compositions comprise from 0.01 to 20%, preferably, from 0.1 to 15% and more preferably, 0.5 to 10% by weight of the coloring agent, when used, relative to the weight of the cosmetic composition.

Preservatives can be incorporated into the present cosmetic compositions as desired to protect against the growth of potentially harmful microorganisms. Cosmetic chemists are familiar with appropriate preservatives and routinely choose them to satisfy preservative tests and product stability tests. Preservative systems should be selected having regard for the use of the composition and possible incompatibilities between the preservatives and other ingredients in the formulation. Exemplary examples of preservatives for cosmetic compositions disclosed herein include, without limitations, iodopropynyl butyl carbamate (IPBC), phenoxyethanol, ethylhexylglycerine, 1,2-octanediol, methyl paraben, propyl paraben, imidazolidinyl urea, sodium dehydroacetate, alkyl esters of para-hydroxybenzoic acid, hydroxyacetophenone, DMDM hydantoin derivatives, climbazole, propionate salts, and a variety of quaternary ammonium compounds. Preservatives are preferably employed in amounts ranging from 0.01 to 2% by weight of the composition.

When making the present cosmetic composition and where the cosmetic composition is in the form of a wash-off or a leave-on composition, the desired ingredients are mixed via conventional methods. If applicable, the oil phase and water phases are separately mixed using standard mixers and mixing blades and heated to a temperature usually from about 22 to about 85°C under atmospheric pressure. Upon reaching the desired temperature, the oil phase is added to the water phase to create an emulsion. Other ingredients in the cosmetic composition are subsequently added and mixed in no particular order.

The cosmetic composition disclosed herein is a composition desirable for topical application to human skin, including leave-on and wash-off products. Preferably, the term encompasses a fluid liquid, and particularly a moisturizer or cleanser rather than a make-up product.

Many types of packaging can be used to store and deliver the composition disclosed herein. The selection of packaging is dependent upon the personal care end-use and the viscosity of the composition itself. As an example, leave-on lotions and creams for skin typically employ plastic containers with an opening at a dispense end covered by an appropriate closure. Conventional closures include flip-top hinged lids, screw-caps and non-aerosol pumps. As another example, appropriate packaging to be used for antiperspirants, deodorants and depilatories include a container with a roller-ball applicator on a dispensing end if the composition is fluid and of a thinner viscosity. If the composition is in a stick format, a container with a propel-repel mechanism wherein the stick is fixed on a platform towards a dispensing orifice is appropriate. If the composition is in an aerosol format, then metallic cans pressurized by a propellant and having a spray nozzle is appropriate. In general, patches, bottles, tubes, roller-ball applicators, squeeze containers or lidded jars are preferred.

The cosmetic compositions will now be described below in the context of specific non-limiting examples to facilitate a greater understanding of the inventive compositions. One of ordinary skill in the art will recognize that variations of the present cosmetic compositions that differ from the examples given may be practiced without deviating from the teachings of the present compositions.

Examples

All samples were made by mixing the mentioned ingredients under conditions of moderate sheer, about 22°C to about 85°C, and atmospheric pressure. All samples were made to target a final pH range of 5.5 to 6.5.

Example 1: Sample formulation

| Sample # | 0 |
|---|---|
| Ingredient | Weight percent (wt%) |
| Deionized water | Balance |
| Chelator | 0.50 |
| Polyquaternium-39 | 1.0 |
| Pearl concentrate/pearlizer | 3.0 |
| Glycerin | 2.0 |
| D-Panthenol (75%) | 1.3 |
| Citric acid | 0.0 |
| Sodium Laureth Sulfate (70%) | 9.1 |
| Decyl glucoside | 5.0 |
| Cocamidopropyl betaine | 6.3 |
| Skin benefit agent | 0.01 |
| Thickening agent | 4.5 |
| PEG-7 Glyceryl Cocoate | 1.0 |
| Neutralizer | 0.50 |
| Preservative | 0.78 |
|  |  |
| Antioxidant | 0.01 |

A cleansing, wash-off composition, hereinafter referred to as Sample 0, was made by combining the above ingredients in accordance with the present cosmetic compositions. The inclusion levels and specific identities of polyol, organic acid, and vitamin $B_5$ compound may be varied to observe the impact of each factor on the mildness, which correlates with enhanced protein and/or lipid integrity in the skin barrier, of the composition on the skin or a model thereof.

Example 2: Control sample with no polyol, organic acid, or vitamin $B_5$ compound

| Sample # | 1 |
|---|---|
| Ingredient | Weight percent (wt%) |
| Deionized water | Balance |
| Chelator | 0.50 |
| Polyquaternium-39 | 1.0 |
| Pearl concentrate/pearlizer | 3.0 |
| Glycerin | 0.0 |

(continued)

| Sample # | 1 |
|---|---|
| Ingredient | Weight percent (wt%) |
| D-Panthenol (75%) | 0.0 |
| Citric acid | 0.0 |
| Sodium Laureth Sulfate (70%) | 9.1 |
| Decyl qlucoside | 5.0 |
| Cocamidopropyl betaine | 6.3 |
| Skin benefit agent | 0.01 |
| Thickening agent | 4.5 |
| PEG-7 Glyceryl Cocoate | 1.0 |
| Neutralizer | 0.50 |
| Preservative | 0.78 |
| Antioxidant | 0.01 |

Sample 1, which comprises 0% by weight of polyol (i.e. glycerin). 0% by weight of organic acid (i.e. organic acid), and 0% by weight of a vitamin $B_5$ compound (i.e., D-Panthenol), was prepared. Sample 1 is to be used as a negative control with which the samples in subsequently disclosed experiments are compared.

Example 3: Samples varying inclusion levels of a vitamin $B_5$ compound

| Sample # | 2 | 3 | 4 | 5 | 6 | 7 |
|---|---|---|---|---|---|---|
| Ingredient | Weight percent (wt%) | | | | | |
| Deionized water | Balanc e | Balanc e | Balance | Balance | Balance | Balance |
| Chelator | 0.50 | 0.50 | 0.50 | 0.50 | 0.50 | 0.50 |
| Polyquaternium-39 | 1.0 | 1.0 | 1.0 | 1.0 | 1.0 | 1.0 |
| Pearl concentrate/pearliz er | 3.0 | 3.0 | 3.0 | 3.0 | 3.0 | 3.0 |
| Glycerin | 0.0 | 0.0 | 0.0 | 0.0 | 0.0 | 0.0 |
| **D-Panthenol (75%)** | 0.7 | 1.3 | 2.7 | 4.0 | 5.3 | 6.7 |
| Citric acid | 0.0010 | 0.0010 | 0.0010 | 0.0010 | 0.0010 | 0.0010 |
| Sodium Laureth Sulfate (70%) | 9.1 | 9.1 | 9.1 | 9.1 | 9.1 | 9.1 |
| Decyl glucoside | 5.0 | 5.0 | 5.0 | 5.0 | 5.0 | 5.0 |
| Cocamidopropyl betaine | 6.3 | 6.3 | 6.3 | 6.3 | 6.3 | 6.3 |
| Skin benefit agent | 0.01 | 0.01 | 0.01 | 0.01 | 0.01 | 0.01 |
| Thickening agent | 4.5 | 4.5 | 4.5 | 4.5 | 4.5 | 4.5 |
| PEG-7 Glyceryl Cocoate | 1.0 | 1.0 | 1.0 | 1.0 | 1.0 | 20 1.0 |
| Neutralizer | 0.50 | 0.50 | 0.50 | 0.50 | 0.50 | 0.50 |
| Preservative | 0.78 | 0.78 | 0.78 | 0.78 | 0.78 | 0.78 |
| Antioxidant | 0.01 | 0.01 | 0.01 | 0.01 | 0.01 | 0.01 |

(continued)

| Sample # | 2 | 3 | 4 | 5 | 6 | 7 |
|---|---|---|---|---|---|---|
| Ingredient | Weight percent (wt%) | | | | | |
| % active D-Panthenol in sample | 0.5 | 1.0 | 2.0 | 3.0 | 4.0 | 5.0 |

Samples 2 to 7 were prepared wherein levels of a vitamin B5 compound, namely D-Panthenol (75% active) as commercially available under the tradename D-Panthenol 75L from DSM Nutritional Products, LLC, were varied.

Example 4: Samples varying inclusion levels of polyol

| Sample # | 8 | 9 | 10 | 11 | 12 |
|---|---|---|---|---|---|
| Ingredient | Weight percent (wt%) 10 | | | | |
| Deionized water | Balance | Balance | Balance | Balance | Balance |
| Chelator | 0.50 | 0.50 | 0.50 | 0.50 | 0.50 |
| Polyquaternium-39 | 1.0 | 1.0 | 1.0 | 1.0 | 1.0 |
| Pearl concentrate/pearliz er | 3.0 | 3.0 | 3.0 | 3.0 | 3.0 |
| **Glycerin** | 1.0 | 2.0 | 3.0 | 4.0 | 5.0 |
| D-Panthenol (75%) | 0.0 | 0.67 | 1.3 | 2.7 | 4.0 |
| Citric acid | 0.0010 | 0.0010 | 0.0010 | 0.0010 | 0.0010 |
| Sodium Laureth Sulfate (70%) | 9.1 | 9.1 | 9.1 | 9.1 | 9.1 |
| Decyl glucoside | 5.0 | 5.0 | 5.0 | 5.0 | 5.0 |
| Cocamidopropyl betaine | 6.3 | 6.3 | 6.3 | 6.3 | 6.3 |
| Skin benefit agent | 0.01 | 0.01 | 0.01 | 0.01 | 0.01 |
| Thickening agent | 4.5 | 4.5 | 4.5 | 4.5 | 4.5 |
| PEG-7 Glyceryl Cocoate | 1.0 | 1.0 | 1.0 | 1.0 | 1.0 |
| Neutralizer | 0.50 | 0.50 | 0.50 | 0.50 | 0.50 |
| Preservative | 0.78 | 0.78 | 0.78 | 0.78 | 0.78 |
| Antioxidant | 0.01 | 0.01 | 0.01 | 0.01 | 0.01 |

Samples 8 to 12 were prepared wherein levels of a polyol, namely glycerin, were varied.

Example 5: Samples varying inclusion levels of both polyol and a vitamin $B_5$ compound

| Sample # | 13 | 14 | 15 | 16 | 17 | 1810 |
|---|---|---|---|---|---|---|
| Ingredient | Weight percent (wt%) | | | | | |
| Deionized water | Balance | Balanc e | Balanc e | Balance | Balance | Balance |
| Chelator | 0.50 | 0.50 | 0.50 | 0.50 | 0.50 | 0.50 |
| Polyquaternium-39 | 1.0 | 1.0 | 1.0 | 1.0 | 1.0 | 1.0 |
| Pearl concentrate/pearliz er | 3.0 | 3.0 | 3.0 | 3.0 | 3.0 | 15 3.0 |
| **Glycerin** | 1.0 | 2.0 | 3.0 | 2.0 | 2.0 | 2.0 |

(continued)

| Sample # | 13 | 14 | 15 | 16 | 17 | 1810 |
|---|---|---|---|---|---|---|
| **Ingredient** | **Weight percent (wt%)** | | | | | |
| **D-Panthenol (75%)** | 1.3 | 1.3 | 1.3 | 0.67 | 2.7 | 4.0 |
| Citric acid | 0.0010 | 0.0010 | 0.0010 | 0.0010 | 0.0010 | 0.0010 |
| Sodium Laureth Sulfate (70%) | 9.1 | 9.1 | 9.1 | 9.1 | 9.1 | 9.1 |
| Decyl glucoside | 5.0 | 5.0 | 5.0 | 5.0 | 5.0 | 5.0 |
| Cocamidopropyl betaine | 6.3 | 6.3 | 6.3 | 6.3 | 6.3 | 6.3 |
| Skin benefit agent | 0.01 | 0.01 | 0.01 | 0.01 | 0.01 | 0.01 |
| Thickening agent | 4.5 | 4.5 | 4.5 | 4.5 | 4.5 | 4.5 |
| PEG-7 Glyceryl Cocoate | 1.0 | 1.0 | 1.0 | 1.0 | 1.0 | 1.0 |
| Neutralizer | 0.50 | 0.50 | 0.50 | 0.50 | 0.50 | 0.50 |
| Preservative | 0.78 | 0.78 | 0.78 | 0.78 | 0.78 | 0.78 |
| Antioxidant | 0.01 | 0.01 | 0.01 | 0.01 | 0.01 | 0.01 |
| | | | | | | |
| % active D-Panthenol in sample | 1.0 | 1.0 | 1.0 | 0.5 | 2.0 | 3.0 |

Samples 13 to 18 were prepared wherein levels of a vitamin $B_5$ compound (i.e., 75% active D-Panthenol) and a polyol (i.e., glycerin) were both varied.

Example 6: Zein tests

Samples are subjected to "Zein tests," a methodology based on the solubilization of a water-insoluble corn polypeptide called Zein by surfactants. Zein is known in the relevant field of the art to resemble proteins present in human skin and hair and the Zein test is therefore appropriately used to model the harshness or mildness of surfactants in various systems when applied topically to skin. Increased solubility of Zein in surfactant solutions correlates to increased skin irritancy, thereby indicating greater harshness of such surfactants on the human skin.

Samples are prepared and 6.25 g of the samples are diluted to 50 g with deionized water and the resulting solution is mixed until uniform. 6 mL of the solution is withdrawn and filtered through a 0.45-micron syringe filter into a vial. This solution is marked to be used as a blank (i.e., a control to correct for any soluble material).

2 g of Zein powder as commercially supplied by Acros Organics is added into the surfactant-containing solution samples and such a resulting mixture is allowed to equilibrate for 1 hour at constant stirring speed (300 rpm). After several minutes of stirring, if all or most of the Zein is dissolved, an additional 1 g of Zein powder is added. Increments of 1 g of Zein is subsequently added into the mixtures until the mixture is fully saturated with Zein (i.e., until there is undissolved Zein floating in the solution). After 1 hour of consistent stirring, the solution settles for 5 minutes. 6 mL of the supernatant solution is withdrawn using a syringe and is filtered through a 0.45-micron syringe filter into a vial. Such samples are marked as test samples to be compared with the blanks.

Each sample is placed in a conventional oven set at 75°C and samples are allowed to dry overnight for approximately 16 hours, allowing for the solvent (i.e., water) to evaporate. The weight of each sample after drying is measured. The remaining percent of Zein dissolved is represented by the equation:

$$(\% \text{ Zein solubilized}) = \% \text{ nonvolatile of sample} - \% \text{ nonvolatile of blank}$$

Table-A:

| Sample # | 0 | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|---|
| % Zein solubilized | 2 | 11 | 10 | 10 | 9 | 9 | 8 | 10 | 10 | 9 | 9 | 9 | 7 | 6 | 6 | 5 | 5 | 4 | 4 |

Results in Table-A are expected from Zein tests for each respective Sample from Sample 0 to 18. A higher the value of % Zein solubilized represents greater protein solubilization of skin proteins and lipids by the surfactants found in the sample and is thus harsher on the skin barrier. Comparatively, a lower value for % Zein solubilized represents a lower protein and lipid solubilization in the skin by surfactants in the sample and is thus milder on the skin barrier. As may be seen in the disclosed sample formulations, all samples are controlled (i.e., consistent) with regards to the surfactant system selected for use. The polyol, organic acid, and vitamin $B_5$ compound in Sample 1, which is made according to the present inventive composition, is expected to attribute to enhanced mildness of the cleansing composition.

**Claims**

1. Use of polyol, organic acid or derivative thereof, and a vitamin $B_5$ compound to cleanse skin and protect and support the integrity of proteins and/or lipids of the skin barrier.

2. Use of polyol, organic acid or derivative thereof, and a vitamin $B_5$ compound for promoting the skin's inherent production of amino acid precursors.

3. The use as claimed in Claim 1 or 2, wherein the polyol comprises glycerin, butylene glycol, sorbitol, propanediol, or a mixture thereof.

4. The use as claimed in any of the preceding claims, wherein the polyol is glycerin, propanediol, or a mixture thereof.

5. The use as claimed in any of the preceding claims, wherein the organic acid or derivative thereof comprises adipic acid, ascorbic acid, aspartic acid, cinnamic acid, citric acid, fumaric acid, glutamic acid, glutaric acid, glycolic acid, hydroxybenzoic acid, hydroxycinnamic acid, lactic acid, maleic acid, malic acid, malonic acid, nicotinic acid, phenylacetic acid, pimelic acid, salicylic acid, sorbic acid, tartaric acid, tartronic acid, tropic acid, and derivatives or mixtures thereof.

6. The use as claimed in any of the preceding claims, wherein the organic acid or derivative thereof is citric acid, a citric acid derivative, lactic acid, a lactic acid derivative, or mixture thereof.

7. The use as claimed in any of the preceding claims, wherein the vitamin $B_5$ compound is selected from the group consisting of pantothenic acid, salts of pantothenic acid, panthenol, derivatives of panthenol, pantolactone, and mixtures thereof.

8. The use as claimed in any of the preceding claims, wherein the vitamin $B_5$ compound is panthenol.

9. The use as claimed in any of the preceding claims, wherein the use is non-therapeutic in nature.

10. The use as claimed in Claim 1, wherein the polyol, organic acid or derivative thereof, and vitamin $B_5$ compound are dispersed in a cosmetically acceptable carrier.

11. The use as claimed in Claim 3, wherein the polyol is 0.1 to 50%, preferably, 0.25 to 40%, more preferably, 0.5 to 30% by weight of the cosmetically acceptable carrier.

12. The use as claimed in Claim 5, wherein the organic acid or derivative thereof is 0.001 to 10%, preferably, 0.002 to 8%, more preferably, 0.003 to 6% by weight of the cosmetically acceptable carrier.

13. The use as claimed in Claim 7, wherein the vitamin $B_5$ compound is 0.001 to 9%, preferably, 0.005 to 7%, and, more preferably, 0.01 to 5% by weight of the cosmetically acceptable carrier.

14. A cosmetic composition as claimed in any of Claims 10 to 13, wherein the cosmetically acceptable carrier is aqueous.

15. A method of topical application to the skin without compromising the integrity of the skin's proteins and/or lipids comprising the steps of:

(a) contacting the skin with a cosmetic composition comprising polyol, organic acid or derivative thereof, and a

vitamin B$_5$ compound; and
(b) washing off or leaving on the composition the skin.

**EP 4 112 039 A1**

Europäisches Patentamt
European Patent Office
Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 492 407 B2 (SUNDBOM NILSSON JOHAN [SE]; LODÉN MARIE [SE] ET AL.) 15 November 2016 (2016-11-15) * column 1, line 15 – line 25; example c3; table 1 * * column 2, line 21 – column 7, line 45 * ----- | 1–15 | INV. A61K8/365 A61K8/34 A61K8/42 A61Q19/00 A61Q19/10 |
| X | DATABASE GNPD [Online] MINTEL; 1 March 2018 (2018-03-01), anonymous: "Hypoallergic Creamy Body Wash Gel", XP055978013, Database accession no. 5491109 * abstract * ----- | 1–10,14, 15 | |
| X | US 2011/159061 A1 (WARREN RAPHAEL [US]) 30 June 2011 (2011-06-30) * paragraph [0018] – paragraphs [0021], [0066]; claims; examples 1-3,5,6,10; table 1 * ----- | 1–15 | |
| X | EBNER FRITZ ET AL: "Topical use of dexpanthenol in skin disorders", AMERICAN JOURNAL OF CLINICAL DERMATOLOGY, ADIS, US, vol. 3, no. 6, 1 January 2002 (2002-01-01) , pages 427-433, XP009181863, ISSN: 1175-0561, DOI: 10.2165/00128071-200203060-00005 * the whole document * ----- | 1,2,7-9, 14,15 | TECHNICAL FIELDS SEARCHED (IPC) A61K A61Q |

-/--

The present search report has been drawn up for all claims

| Place of search | Date of completion of the search | Examiner |
|---|---|---|
| Munich | 7 November 2022 | Loloiu, Teodora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

Application Number

EP 22 17 9970

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAEHNHARDT DOROTHEE ET AL: "The Influence of Two Different Foam Creams on Skin Barrier Repair of Foot Xerosis: A Prospective, Double-Blind, Randomised, Placebo-Controlled Intra-Individual Study", SKIN PHARMACOLOGY AND PHYSIOLOGY, vol. 29, no. 5, 1 January 2016 (2016-01-01), pages 266-272, XP055920868, CH ISSN: 1660-5527, DOI: 10.1159/000448690 * Materials and Methods, Results * ----- | 1-4,7-9, 14,15 | |
| X | PROKSCH E ET AL: "Dexapanthenol enhances skin barrier repair and reduces inflammation after sodium lauryl sulphate-induced irritation", THE JOURNAL OF DERMATOLOGICAL TREATMENT, vol. 13, no. 4, 19 January 2002 (2002-01-19), pages 173-178, XP055920901, UK ISSN: 0954-6634, DOI: 10.1080/09546630212345674 Retrieved from the Internet: URL:http://dx.doi.org/10.1080/095466302123 45674> * the whole document * ----- -/-- | 1,2,7-9, 14,15 | TECHNICAL FIELDS SEARCHED (IPC) |

The present search report has been drawn up for all claims

| Place of search | Date of completion of the search | Examiner |
|---|---|---|
| Munich | 7 November 2022 | Loloiu, Teodora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 22 17 9970

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | PURNAMAWATI SCHANDRA ET AL: "The Role of Moisturizers in Addressing Various Kinds of Dermatitis: A Review", CLINICAL MEDICINE AND RESEARCH, vol. 15, no. 3-4, 1 December 2017 (2017-12-01), pages 75-87, XP055920863, US ISSN: 1539-4182, DOI: 10.3121/cmr.2017.1363 Retrieved from the Internet: URL:https://www.ncbi.nlm.nih.gov/pmc/artic les/PMC5849435/pdf/0150075.pdf> ----- | | |

TECHNICAL FIELDS
SEARCHED     (IPC)

The present search report has been drawn up for all claims

| Place of search | Date of completion of the search | Examiner |
|---|---|---|
| Munich | 7 November 2022 | Loloiu, Teodora |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

page 3 of 3

## ANNEX TO THE EUROPEAN SEARCH REPORT
## ON EUROPEAN PATENT APPLICATION NO.

EP 22 17 9970

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2022

| Patent document cited in search report | | Publication date | Patent family member(s) | | Publication date |
|---|---|---|---|---|---|
| US 9492407 | B2 | 15-11-2016 | AU | 2012254214 B2 | 20-10-2016 |
| | | | CA | 2835874 A1 | 15-11-2012 |
| | | | CN | 103717212 A | 09-04-2014 |
| | | | DK | 2522342 T3 | 14-07-2014 |
| | | | EA | 201391592 A1 | 30-04-2014 |
| | | | EP | 2522342 A1 | 14-11-2012 |
| | | | ES | 2475840 T3 | 11-07-2014 |
| | | | JP | 6062928 B2 | 18-01-2017 |
| | | | JP | 2014527024 A | 09-10-2014 |
| | | | US | 2014073613 A1 | 13-03-2014 |
| | | | WO | 2012154122 A2 | 15-11-2012 |
| | | | ZA | 201308459 B | 25-02-2015 |
| US 2011159061 | A1 | 30-06-2011 | BR | 112012016098 A2 | 31-05-2016 |
| | | | BR | 112012016100 A2 | 31-05-2016 |
| | | | CA | 2784031 A1 | 07-07-2011 |
| | | | CA | 2784190 A1 | 07-07-2011 |
| | | | CN | 102695482 A | 26-09-2012 |
| | | | CN | 102740816 A | 17-10-2012 |
| | | | EP | 2519205 A1 | 07-11-2012 |
| | | | EP | 2519206 A1 | 07-11-2012 |
| | | | EP | 2519213 A1 | 07-11-2012 |
| | | | JP | 2013515589 A | 09-05-2013 |
| | | | JP | 2013515778 A | 09-05-2013 |
| | | | KR | 20120093393 A | 22-08-2012 |
| | | | KR | 20120093394 A | 22-08-2012 |
| | | | RU | 2012119346 A | 10-02-2014 |
| | | | RU | 2012119353 A | 10-02-2014 |
| | | | US | 2011159061 A1 | 30-06-2011 |
| | | | US | 2011160688 A1 | 30-06-2011 |
| | | | WO | 2011082025 A1 | 07-07-2011 |
| | | | WO | 2011082027 A1 | 07-07-2011 |
| | | | WO | 2011082151 A1 | 07-07-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030211068
- US 8628758 B
- US 10973749 B
- WO 199947114 A
- WO 2021082683 A
- US 5393466 A, Ilardi
- US 5389279 A, Au
- US 5009814 A, Kelkenberg